# EUROPEAN PATENT APPLICATION

(11) **EP 3 878 402 A1**
(43) Date of publication of application: **15.09.2021**
(21) Application number: 19882346.0
(22) Date of filing: 26.09.2019
(51) Int. Cl.: A61C 17/02, A61L 2/00, A61M 11/00, C02F 1/66, C02F 1/467

(54) **ORAL CLEANING APPARATUS**

(30) Priority: 09.11.2018 KR 20180136981; 26.04.2019 KR 20190049366
(71) Applicant: H&Care Co., Ltd., Wonju-si, Gangwon-do 26361 (KR)
(72) Inventor: KIM, Yong Hwan, Yeoju-si Gyeonggi-do 12637 (KR)
(74) Representative: HGF
(86) International application number: PCT/KR2019/012500
(87) International publication number: WO 2020/096200

(57) **Abstract**

One purpose of the present invention is to provide an oral cleaning apparatus having a beautiful appearance and reducing fatigue when used. The present invention provides an oral cleaning apparatus. According to an embodiment, an oral cleaning apparatus comprises: a body having a donut shape with a hole formed therein when viewed from the side, the body including a front portion provided in front of the hole and a rear portion provided behind the hole; a nozzle tip provided at the front portion of the body and discharging cleaning water to the outside; a water tank provided in the body and storing the cleaning water; a pump for pumping the cleaning water, which is stored in the water tank, to the nozzle tip; and a power source for supplying power to the pump, wherein the center of gravity of the body may be provided biased toward the front portion.

## Description

### TECHNICAL FIELD

The present invention relates to an oral cleaning apparatus with improved portability and usability.

### BACKGROUND ART

An oral cleaning apparatus may clean foreign substances stuck between teeth by discharging cleaning water through a nozzle tip. The oral cleaning apparatus cleans teeth in an oral cavity by injecting water, which is forcedly pumped by a pump disposed in a body, with strong hydraulic pressure through the nozzle tip. The oral cleaning apparatus may be simply and quickly used and also effectively clean teeth in comparison with a toothbrush and a dental floss.

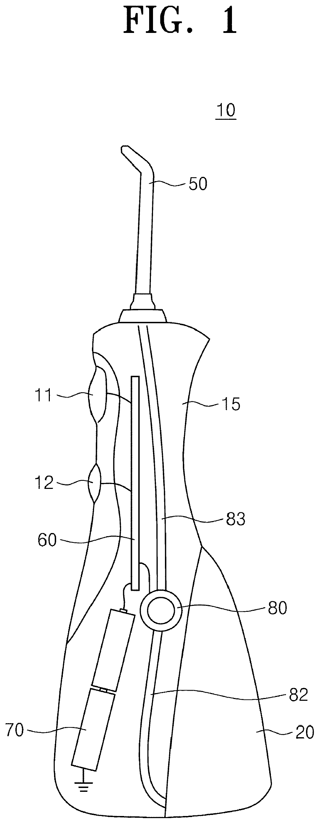

The oral cleaning apparatus is provided for portable use. FIG. 1 is a typical portable oral cleaning apparatus 10. Referring to FIG. 1, the typical portable oral cleaning apparatus 10 includes a nozzle tip 50, a circuit board 60, a power source 70, a pump 80, and a water tank 20. The water tank 20 and the power source 70 are provided at a bottom portion of a body. Cleaning water stored in the water tank 20 is discharged to the nozzle tip 50 by using the pump 80 connected with water discharge pipes 82 and 83. A gripping area 15 is provided at an upper portion of the body, and switches 11 and 12 are provided at the gripping area 15.

This typical portable oral cleaning apparatus 10 is inconvenient as a weight of the cleaning water is added to a weight of the apparatus.

### DISCLOSURE OF THE INVENTION

### TECHNICAL PROBLEM

The present invention provides a portable oral cleaning apparatus.

The present invention also provides an oral cleaning apparatus that is convenient for use.

The present invention also provides an oral cleaning apparatus having a beautiful appearance and reducing fatigue when used.

The object of the present invention is not limited to the aforesaid, but other objects not described herein will be clearly understood by those skilled in the art from descriptions below.

### TECHNICAL SOLUTION

The present invention provides an oral cleaning apparatus. According to an embodiment, an oral cleaning apparatus includes: a body having a donut shaped side surface with a hole defined therein and including a front portion provided in front of the hole and a rear portion provided behind the hole; a nozzle tip provided at the front portion of the body to discharge cleaning water to the outside; a water tank provided in the body to store the cleaning water; a pump configured to pump the cleaning water, which is stored in the water tank, to the nozzle tip; and a power source configured to supply power to the pump, and a center of gravity of the body may be biased toward the front portion.

According to an embodiment, a gripping area may be provided at the front portion.

According to an embodiment, a center of gravity of the body may be biased toward the gripping area.

According to an embodiment, the water tank may be provided at the rear portion, and the pump and the power source may be built in the front portion.

According to an embodiment, a sterilization module may be provided in the water tank.

According to an embodiment, a holding surface may be defined on a bottom surface of the body.

According to an embodiment, the hole of the body may have a longitudinal length greater than a transverse width thereof.

According to an embodiment, the nozzle tip may be coupled to a top surface of the front portion.

### ADVANTAGEOUS EFFECTS

The oral cleaning apparatus according to an embodiment of the present invention is portable.

Also, the oral cleaning apparatus according to an embodiment of the present invention is convenient for use.

Also, the oral cleaning apparatus according to an embodiment of the present invention has the beautiful appearance and reduces fatigue when used.

The object of the present invention is not limited to the aforesaid, but other objects not described herein will be clearly understood by those skilled in the art from descriptions below.

### BRIEF DESCRIPTION OF THE DRAWINGS

FIG. 1 is a typical portable oral cleaning apparatus.
FIG. 2 is a front perspective view of a first embodiment.
FIG. 3 is a rear perspective view of the first embodiment.
FIG. 4 is a schematic cross-sectional view illustrating an inner configuration of the first embodiment.
FIG. 5 is a bottom view of the first embodiment.
FIG. 6 is a perspective view of a holding member 1000 according to the first embodiment of the present invention.
FIG. 7 is a cross-sectional view illustrating an inner configuration of an oral cleaning apparatus 200 according to a second embodiment.
FIG. 8 is a cross-sectional view illustrating an inner configuration of an oral cleaning apparatus 300 according to a third embodiment.
FIG. 9 is a cross-sectional view illustrating an inner configuration of an oral cleaning apparatus 400 according to a fourth embodiment.

### MODE FOR CARRYING OUT THE INVENTION

Hereinafter, preferred embodiments of the present invention will be described in detail with reference to the accompanying drawings in such a manner that the technical idea of the present invention may easily be carried out by a person with ordinary skill in the art to which the invention pertains. The present invention may, however, be embodied in different forms and should not be construed as limited to the embodiments set forth herein. Moreover, when the preferred embodiments of the present invention are described in detail, detailed descriptions related to well-known functions or configurations will be ruled out in order not to unnecessarily obscure subject matters of the present invention. Also, portions having similar functions and effects may be expressed with the same reference symbol throughout.

Furthermore, when it is described that one comprises (or includes or has) some elements, it should be understood that it may comprise (or include or has) only those elements, or it may comprise (or include or have) other elements as well as those elements if there is no specific limitation. The meaning of 'include' or 'comprise' specifies a property, a fixed number, a step, an operation, an element, a component, or a combination thereof but does not exclude other properties, fixed numbers, steps, operations, elements, components, combinations thereof.

The terms of a singular form may include plural forms unless referred to the contrary. Thus, in the drawings, the shapes and sizes of elements may be exaggerated for clarity.

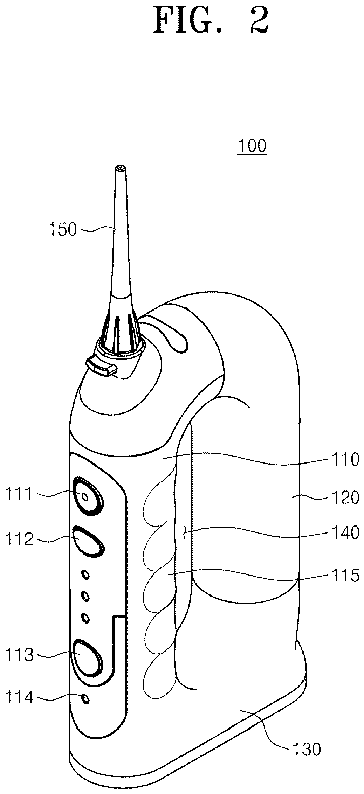

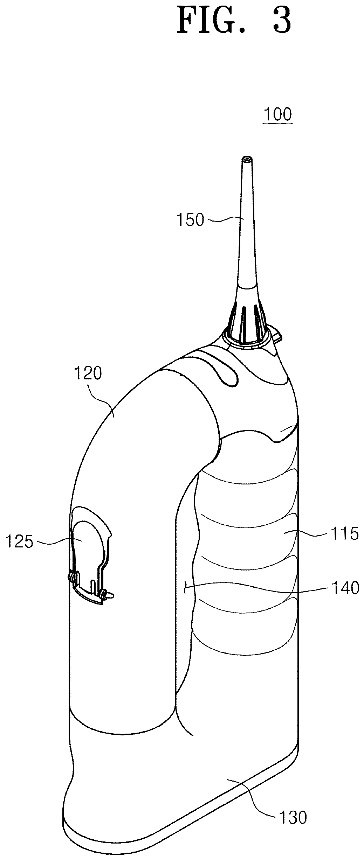

FIG. 2 is a front perspective view of a first embodiment, and FIG. 3 is a rear perspective view of the first embodiment. The first embodiment will be explained with reference to FIGS. 2 and 3.

An oral cleaning apparatus 100 according to the first embodiment includes a body and a nozzle tip 150.

The body has a donut shaped side surface with a hole 140 defined therein. In this specification, the donut shape, as a shape having a penetrated inside, represents that the hole 140 is defined in a rectangular shape or an elliptical shape instead of being limited to a circular shape. The hole 140 may have a longitudinal length greater than a transverse width thereof.

In the body, a front thereof is defined as a front portion 110, and a rear thereof is defined as a rear portion 120 by using the hole 140 as a center. The body has a center of gravity, which is biased toward the front portion 110.

A gripping area 115 is provided in the front portion 110 of the body. A user may grip the front portion 110 by hands to lift the oral cleaning apparatus 100. The center of gravity of the body may be concentrated on the gripping area 115.

Switches 111, 112, and 113 for controlling operations may be provided in front of the gripping area 115. According to an example, a first switch turns power on/off. According to an example, a second switch 112 controls hydraulic pressure. According to an example, a third switch 112 controls an operation of a sterilization module 190, which will be described later. Also, a lamp 114 for providing residual power information of a power source 170 to the user may be provided at the front portion 110.

A nozzle tip 150 is coupled to a top surface of the front portion 110. Although not shown, the nozzle tip 150 may have a water discharge end that is bent toward a front side thereof. The nozzle tip 150 may be separated from the body. The nozzle tip 150 may be replaced by another nozzle tip (not shown). For example, an old nozzle tip may be removed when polluted while used, and a new nozzle tip may be coupled.

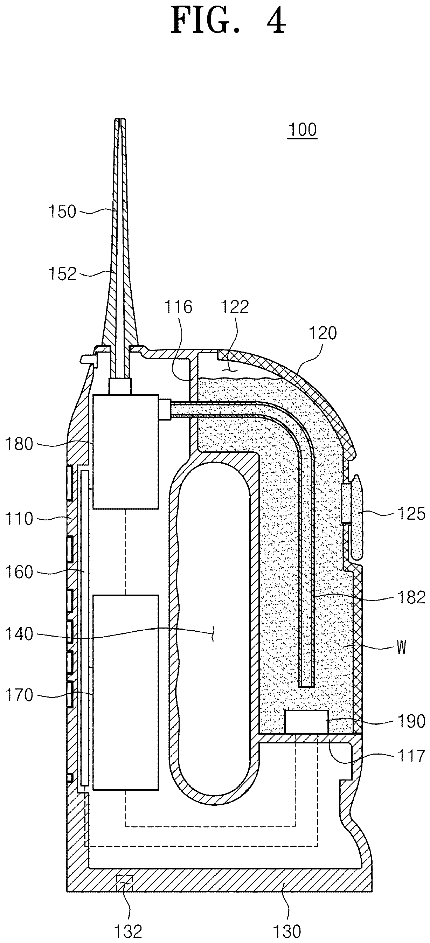

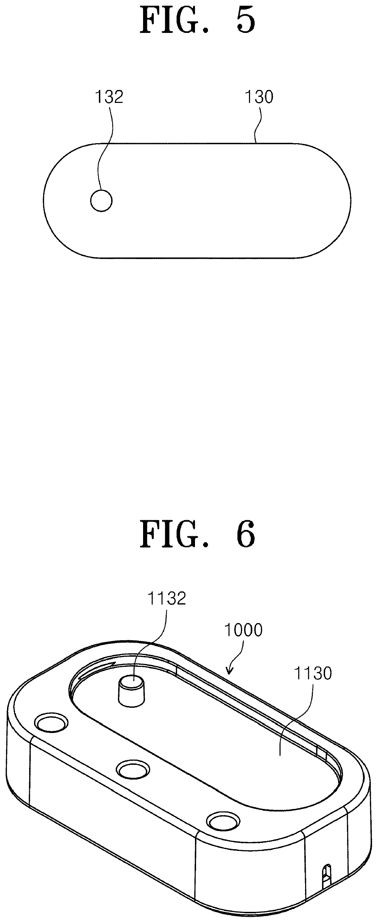

FIG. 4 is a schematic cross-sectional view illustrating an inner configuration of the first embodiment, and FIG. 5 is a bottom view of the first embodiment. A configuration of the oral cleaning apparatus 100 will be explained with reference to FIGS. 4 and 5.

In an example, a pump 180, a circuit board 160, and a power source 170 may be built in the front portion 110 of the body. A water tank is provide at the rear portion 120. Hereinafter, the rear portion and the water tank use the same reference numeral. However, a feature of using the same reference numeral for the rear portion and the water tank of the body is for convenience of description, but does not represent the components are the same as each other.

Cleaning water W is stored in the water tank 120. A water supply part 125 that is able to be opened and closed may be provided to the water tank 120. A housing of the water tank 120 may be made of a transparent material so as to observe the cleaning water. The center of gravity of the body when water is fully filled in the water tank 120 may be biased toward the rear portion 120.

The sterilization module 190 is provide to the water tank 120. The sterilization module 190 may be an electrolysis module that electrolyzes the cleaning water W. As the cleaning water W is provided as water containing an electrolyte, a hydrogen may be generated when electrolyzed. The electrolysis module uses underwater discharge generated in a positive electrode and a negative electrode. This is disclosed in the related art such as KR10-1719681B1 and KR10-1740507B1, which relates to underwater discharge. The hydrogen may be generated when the cleaning water is underwater discharged. Thus, the cleaning water may be hydrogen water.

A second partition wall 186 is disposed at the rear portion of the body to support the sterilization module 190. The second partition wall 186 may be provided in a direction horizontal to the ground adjacent to a bottom surface of the rear portion 120.

The pump 180 pumps the cleaning water W stored in the water tank 120 to the nozzle tip 150. The water tank 120 and the nozzle tip 150 are connected by a connection pipe. The pump 180 is provided in the middle of the connection pipe and provides force for discharging the cleaning water W to the outside of the nozzle tip 150. According to an example, the pump 180 may be provided as a piston module. The piston module may be driven by a motor (not shown).

A water discharge pipe 182 is inserted into the water tank 120. The front portion 110 and the rear portion 120 of the body 110 are divided by a first partition wall 116. The water discharge pipe 182 passes through the first partition wall 116. The discharge line 182 has one end connected to a discharge pump (not shown) and the other end connected to the lower portion of the chamber 120. The water discharge pipe 182 extends from an upper portion to a bottom surface of the water tank 120. As the water discharge pipe 182 is connected to the upper portion of the water tank 120, a path from the water tank 120 to the nozzle tip 150 may have a short distance.

The circuit board 160 includes a control part. The circuit board 160 may control an operation of the pump 180 and the sterilization module 190. The circuit board 160 may be connected with switches, and an operation of the circuit board 160 may be controlled from the outside.

The power source 170 supplies power to the pump 180, the circuit board 160, and the sterilization module 190. The power source 170 is disposed at the front portion 110 of the body. Specifically, the power source 170 is disposed below the pump 180. The power source 170 is provided as a rechargeable battery. For example, the power source 170 may be a lithium-ion battery. The power source 170 may be charged by receiving power from the outside. The body may further include a power receiving coil (not shown).

A holding surface 130 is provided at the bottom surface of the body. A coupling groove 132 may be defined at the holding surface 130. The coupling groove 132 is coupled with a coupling projection 1132 provided on a holding member 1000 that will be described later. The coupling projection 1132 and the coupling groove 132 serve as a guide when the oral cleaning apparatus is coupled with the holding member 1000. According to an embodiment, the holding member 1000 may be provided as a charging pad. The holding member 1000 may include a power transmitting coil (not shown). When the oral cleaning apparatus 100 is fixed at a regular position of the holding member 1000, the oral cleaning apparatus 100 may be charged by a contactless manner by interaction between the power receiving coil (not shown) and the power transmitting coil (not shown).

FIG. 6 is a perspective view of the holding member 1000 according to the first embodiment. Referring to FIG. 6, a holding groove 1130 to which the holding surface 130 of the oral cleaning apparatus 100 is insertable may be defined in the holding member 1000. The coupling projection 1132 coupled with the coupling groove 132 of the oral cleaning apparatus 100 is provided at the holding groove 1130. The coupling projection 1132 and the coupling groove 132 serve as a guide when the oral cleaning apparatus is coupled with the holding member 1000.

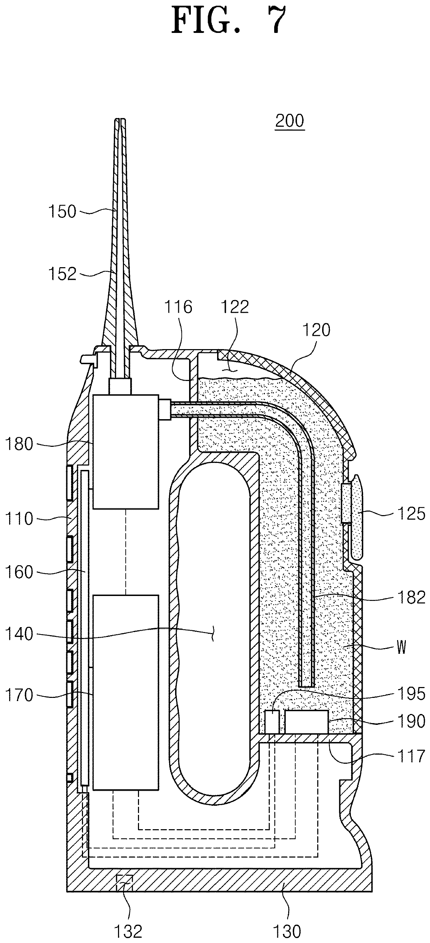

FIG. 7 is a cross-sectional view illustrating an inner configuration of an oral cleaning apparatus 200 according to a second embodiment. Referring to FIG. 7, a pH sensor 195 may be further provided in a water tank 120 of the oral cleaning apparatus 200. The pH sensor 195 may sense pH of cleaning water W and transmit sensed pH information to a control part. The control part may control an operation of the sterilization module 190 based on the received pH information. According to an embodiment, the pH sensor 195 may real-time sense the pH of the cleaning water W. As the pH of the cleaning water W is monitored by the pH sensor 195, the sterilization module 190 may be effectively controlled. According to an example, the power of the power source 170 may be saved by effectively controlling the operation of the sterilization module 190.

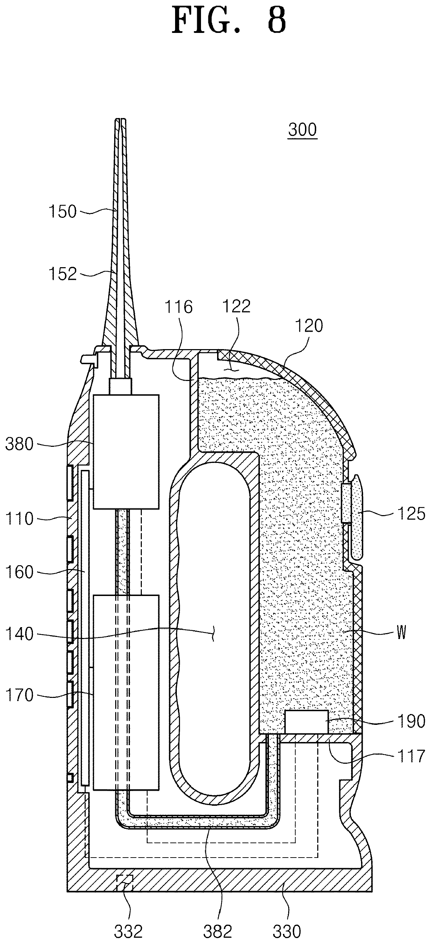

FIG. 8 is a cross-sectional view illustrating an inner configuration of an oral cleaning apparatus 300 according to a third embodiment. Referring to FIG. 8, a water discharge pipe 382 is connected from a bottom surface of a water tank 120 to a nozzle tip 150. The water discharge pipe 382 is connected to a pump 180 and discharges cleaning water through the nozzle tip 150.

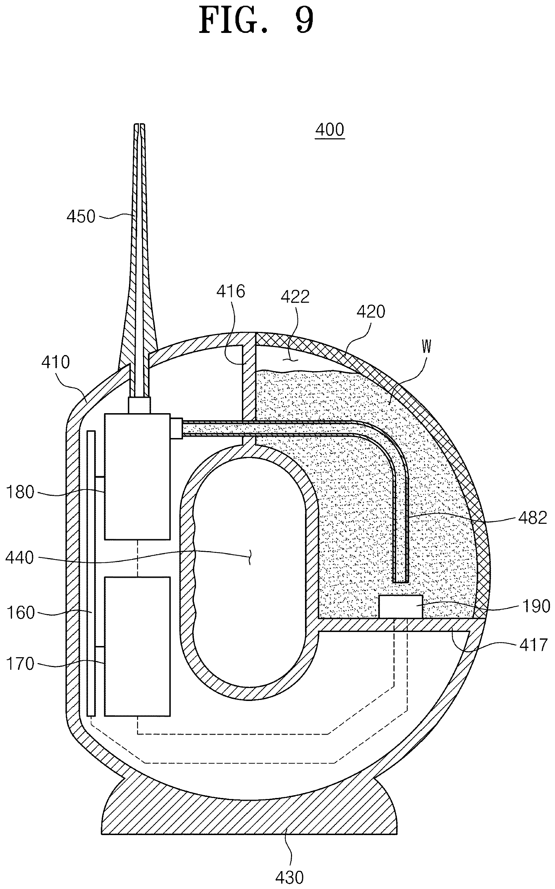

FIG. 9 is a cross-sectional view illustrating an inner configuration of an oral cleaning apparatus 400 according to a fourth embodiment. Referring to FIG. 8, a front portion 410, a rear portion 420, and a holding surface 430, which have different shapes from those of the first embodiment, are provided. A body of the oral cleaning apparatus 400 according to the fourth embodiment may have a circular donut shape. As an appearance has a shape similar to a snail or a donut, a sense of pleasure may be added to oral cleaning.

According to the above-described embodiments, although the pump and the power source are arranged at the front portion, the arrangement may be varied when a component having an additional function is added.

According to the above-described embodiments, although the power source is provided below the pump, the power source may be provided above the pump, and the power source and the pump may be spaced a long distance from each other. This may be changed according to a design.

According to the above-described embodiments, although the water tank is coupled to the body, the water tank may be provided separably from the body.

The above detailed description exemplifies the present invention. Further, the above contents just illustrate and describe preferred embodiments of the present invention and the present invention can be used under various combinations, changes, and environments. That is, it will be appreciated by those skilled in the art that substitutions, modifications and changes may be made in these embodiments without departing from the principles and spirit of the general inventive concept, the scope of which is defined in the appended claims and their equivalents. The above-mentioned embodiments are used to describe a best mode in implementing the technical idea of the present invention, and various modifications required in a detailed application field and usage of the present invention can be made. Therefore, the detailed description of the present invention does not intend to limit the present invention to the disclosed embodiments. Further, the appended claims should be appreciated as a step including even another embodiment.

### [Description of reference numerals]

100, 200, 300, 400: Oral cleaning apparatus
1000: Holding member

## Claims

1. An oral cleaning apparatus comprising:
a body having a donut shaped side surface with a hole defined therein and comprising a front portion provided in front of the hole and a rear portion provided behind the hole;
a nozzle tip provided at the front portion of the body to discharge cleaning water to the outside;
a water tank provided in the body to store the cleaning water;
a pump configured to pump the cleaning water, which is stored in the water tank, to the nozzle tip; and
a power source configured to supply power to the pump.

2. The oral cleaning apparatus of claim 1, wherein a center of gravity of the body is biased toward the front portion.

3. The oral cleaning apparatus of claim 1, wherein a gripping area is provided at the front portion.

4. The oral cleaning apparatus of claim 3, wherein a center of gravity of the body is biased toward the gripping area.

5. The oral cleaning apparatus of claim 1, wherein the water tank is provided at the rear portion, and
the pump and the power source are built in the front portion.

6. The oral cleaning apparatus of claim 1, wherein a sterilization module is provided in the water tank.

7. The oral cleaning apparatus of claim 1, wherein a holding surface is defined on a bottom surface of the body.

8. The oral cleaning apparatus of claim 1, wherein the hole of the body has a longitudinal length greater than a transverse width thereof.

9. The oral cleaning apparatus of claim 1, wherein the nozzle tip is coupled to a top surface of the front portion.

10. The oral cleaning apparatus of claim 1, further comprising a water discharge pipe disposed in the water tank and having one end connected to the pump and the other end disposed in the water tank,
wherein the front portion and the rear portion of the body are divided by a first partition wall, and
the water discharge pipe passes through the first partition wall to extend up to a lower portion of the water tank.

11. The oral cleaning apparatus of claim 6, wherein a second partition wall configured to support the sterilization module is provided at the rear portion of the body.
